Europäisches Patentamt

(19) European Patent Office

Office européen des brevets

(11) **EP 0 643 585 B1**

(12) **EUROPEAN PATENT SPECIFICATION**

(45) Date of publication and mention
of the grant of the patent:
**15.12.1999 Bulletin 1999/50**

(21) Application number: **93910775.1**

(22) Date of filing: **23.04.1993**

(51) Int. Cl.$^6$: **A61K 38/55**

(86) International application number:
**PCT/US93/03860**

(87) International publication number:
**WO 93/24143 (09.12.1993 Gazette 1993/29)**

(54) **The use of Lipoprotein Associated Coagulation Inhibitor (LACI) for the preparation of a medicament for the treatment of acute or chronic inflammation.**

Zusammensetzungen enthaltend Lipoprotein-Associated-Coagulation-Inhibitor (LACI) zur Herstellung eines Medikaments zur Behandlung von akuten oder chronischen Enzündungen

Utilisation de l'inhibiteur de Coagulation Associé aux Lipoproteines (LACI) pour l'obtention d'un médicament destiné au traitement de l'inflammation aigue ou chronique.

(84) Designated Contracting States:
**AT BE CH DE DK ES FR GB GR IE IT LI LU MC NL PT SE**

(30) Priority: **01.06.1992 US 891947**
**13.01.1993 US 4505**

(43) Date of publication of application:
**22.03.1995 Bulletin 1995/12**

(73) Proprietor: **CHIRON CORPORATION**
**Emeryville, California 94608-2916 (US)**

(72) Inventor: **CREASEY, Abla, A.**
**Piedmont, CA 94611 (US)**

(74) Representative:
**Goldin, Douglas Michael et al**
**J.A. KEMP & CO.**
**14 South Square**
**Gray's Inn**
**London WC1R 5LX (GB)**

(56) References cited:
**WO-A-91/02753**

**Description**

The present invention provides the use of lipoprotein associated coagulation inhibitor (LACI) in the manufacture of a medicament for prophylactically and therapeutically treating acute and chronic inflammation, sepsis and septic shock.

Background of the Invention

Sepsis and its sequela septic shock remain among the most dreaded complications after surgery and in critically ill patients. The Center for Disease Control ranks septicemia as the 13th leading cause of death in the United States (see MMWR, 1987, 39:31 and US Dept. of Health and Human Services, 37:7, 1989), and the 10th leading cause of death among elderly Americans (see MMWR, 1987, 39:777). The incidence of these disorders is increasing, and mortality remains high. Estimates of the total cost of caring for patients with septicemia range from $5 billion to $10 billion annually (see MMWR, 1987, 39:31). Death can occur in 40% to 60% of the patients. This percentage has not seen any improvement over the past 20 years.

The incidence of blood borne gram-positive and gram-negative infections that can lead to septic shock occur approximately equally. Septic shock is characterized by inadequate tissue perfusion, leading to insufficient oxygen supply to tissues, hypotension and oliguria. Septic shock occurs because bacterial products, principally LPS, react with cell membranes and components of the coagulation, complement, fibrinolytic, bradykinin and immune systems to activate coagulation, injure cells and alter blood flow, especially in the microvasculature. Microorganisms frequently activate the classic complement pathway, and endotoxin activates the alternate pathway. Complement activation, leukotriene generation and the direct effects of endotoxin on neutrophils lead to accumulation of these inflammatory cells in the lungs, release of the enzymes and production of toxic oxygen radicals which damage the pulmonary endothelium and initiate the acute respiratory distress syndrome (ARDS). ARDS is a major cause of death in patients with septic shock and is characterized by pulmonary congestion, granulocyte aggregation, hemorrhage and capillary thrombi.

Activation of the coagulation system results in thrombin generation and platelet thrombi formation in the microcirculation of many tissues. The pathogenesis of this syndrome involves the activation of the intrinsic coagulation system by Factor XII, as well as activation of the extrinsic pathway through upregulation of tissue factor. Activated Factor XII initiates the intrinsic coagulation cascade and eventually fibrinogen is converted to fibrin and clotting occurs. Uncontrolled activation of coagulation, usually accompanied by shock, will result in thrombosis and consumption of clotting Factors II, V, and VIII. Some common complications of disseminated intravascular coagulation are severe clinical bleeding, thrombosis, tissue ischaemia and necrosis, hemolysis and organ failure.

At the same time, as coagulation is apparently initiated by endotoxin, countervening mechanisms also appear to be activated by clotting, namely activation of the fibrinolytic system. Activated Factor XII converts plasminogen proactivator to plasminogen activator which subsequently converts plasminogen to plasmin thereby mediating clot lysis. The activation of plasma fibrinolytic systems may therefore also contribute to bleeding tendencies.

Endotoxemia is associated with an increase in the circulating levels of tissue plasminogen activator inhibitor (PAI). This inhibitor rapidly inactivates tissue plasminogen activator (TPA), thereby hindering its ability to promote fibrinolysis through activation of plasminogen to plasmin. Impairment of fibrinolysis may cause fibrin deposition in blood vessels, thus contributing to the disseminated intravascular coagulation associated with septic shock.

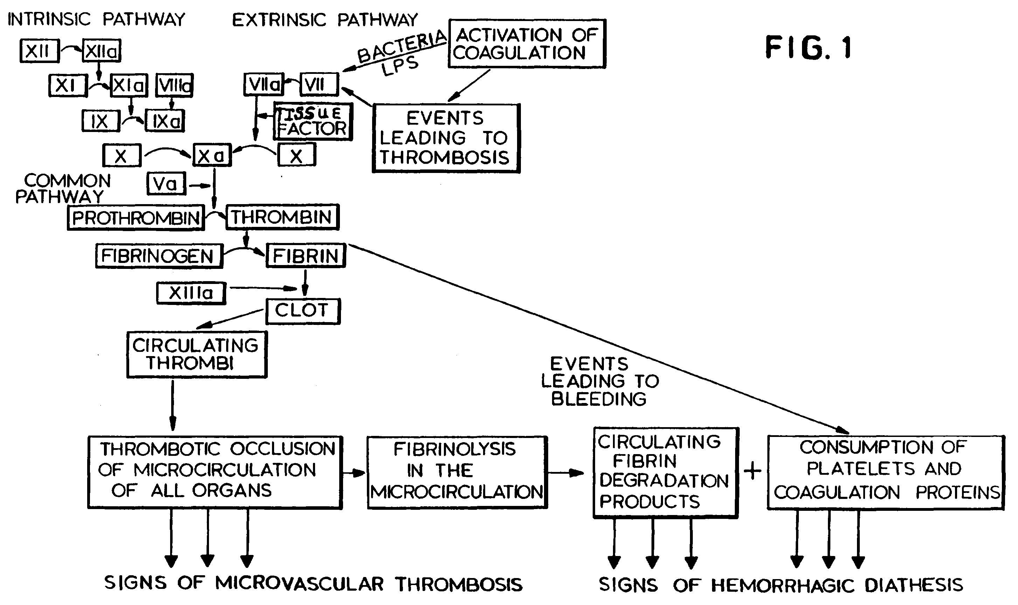

Disseminated intravascular coagulation (DIC) is a coagulopathic disorder that occurs in response to invading microorganisms characterized by widespread deposition of fibrin in small vessels. The initiating cause of DIC appears to be the release of thromboplastin (tissue factor) into the circulation. During this process, there is a reduction in fibrinogen and platelets, and a rise in fibrin split products resulting in fibrin deposition in blood vessels. The sequence of events that occur during DIC are described in Figure 1. The patients either suffer from thrombosis or hemorrhage depending on the extent of exhaustion of the coagulation protease inhibitors during the disease process. Part of the regulation of the coagulation cascade depends on the rate of blood flow. When flow is decreased, as it is in DIC and sepsis, the problems are magnified. DIC (clinically mild to severe form) is thought to occur with high frequency in septic shock patients and several other syndromes such as head trauma and bums, obstetric complications, transfusion reactions, and cancer. A recent abstract by Xoma Corporation indicates that DIC was present on entry in 24% of septic patients (Martin et al., 1989, Natural History in the 1980s, Abstract No. 317, ICAAC Meeting, Dallas). Furthermore, the abstract describes that DIC and acute respiratory distress syndrome were the variables most predictive of death by day 7 (risk ratios 4 and 2.3). The cascade of events that lead to release of tissue factor into circulation and sepsis is very complex. Various cytokines are released from activated monocytes, endothelial cells and others; these cytokines include tumor necrosis factor (TNF), interleukin 1 (IL-1) (which are known to up-regulate tissue factor expression), interleukin 6 (IL-6), gamma interferon (IFN-γ), interleukin 8 (IL-8), and others. The complement cascade is also activated as demonstrated by the rise in C3a and C5a levels in plasma of septic patients. Consequently, an agent that will treat coagulation without affecting the expression of tissue factor or its activity will not necessarily be effective to treat sepsis.

There are currently no satisfactory interventions for the prevention or treatment of sepsis or DIC. Heparin is the most commonly used anticoagulant in DIC. However, it has been controversial because it can induce bleeding and worsen the patient's condition. Other attempts to treat sepsis using an anticoagulant have also been difficult. As shown in Taylor et al., 1991, Blood, 78:364-368, warfarin and heparin are mentioned as two anticoagulants that are used to treat DIC in sepsis, but neither are the ideal drugs. Additionally, Taylor et al. show that a new drug DEGR-Xa, a factor Xa antagonist, can inhibit DIC, however, this drug failed to block the lethal effects of sepsis. Consequently, it is evident that an agent which may interrupt the coagulation pathway is not necessarily effective as an inhibitor of septic shock. Therefore, there is a need for a composition that will inhibit the lethal effects of sepsis.

Summary of the Invention

The present invention provides use of lipoprotein associated coagulation inhibitor (LACI) or a fragment or hybrid thereof which maintains anti-inflammatory activity in the manufacture of a medicament for use in a prophylactic or therapeutic method of treating acute or chronic inflammation.

The invention may therefore be used to provide a method for prophylactically and therapeutically treating syndromes associated with acute or chronic inflammation where activation of Factor VII, Xa and tissue factor expression are involved, such as sepsis and septic shock comprising administering an effective amount of lipoprotein associated coagulation inhibitor (LACI). The LACI may be used in combination with an additional agent, such as an antibiotic, monoclonal antibody, cytokine or complement inhibitor in a combined preparation for use in the treatment of sepsis.

The administration of LACI may be used to treat acute or chronic inflammation in which TNF, IL-1, IL-6 or other cytokines up regulate tissue factor. Preferably, LACI is intravenously administered at a dose from 1 μg/kg to 20 mg/kg, more preferably from 20 μg/kg to 10 mg/kg, most preferably from 1 to 7 mg/kg. LACI is preferably administered with an additional agent to treat sepsis and septic shock, such as an antibiotic.

Among other things, it has been surprisingly discovered that a compound known for its anti-coagulant properties, can also attenuate the immune response and serve as a treatment for sepsis and septic shock. This was surprising in view of the findings of Warr et al., 1990, Blood, 75:1481-1489 and Taylor et al., 1991, Blood, 78:364-368.

Brief Description of the Drawings and Tables

Figure 1 shows the complex pathways involved in Sepsis and septic shock. The intrinsic and extrinsic pathways are included. Signs of microvascular thrombosis include: (1) neurologic: multifocal, delerium, coma; (2) skin: focal ischemia, superficial gangrene; (3) renal: oliguria, azotemia, cortical necrosis; (4) pulmonary: acute respiratory distress syndrome; and (5) gastrointestinal: acute ulceration. Signs of hemorrhagic diathesis include: (1) neurologic: intracerebral bleeding; (2) skin: petechiae, ecchymoses, venepuncture oozing; (3) renal: hematuria; (4) mucous membranes: epistaxis, gingival oozing; and (5) gastrointestinal: massive bleeding.

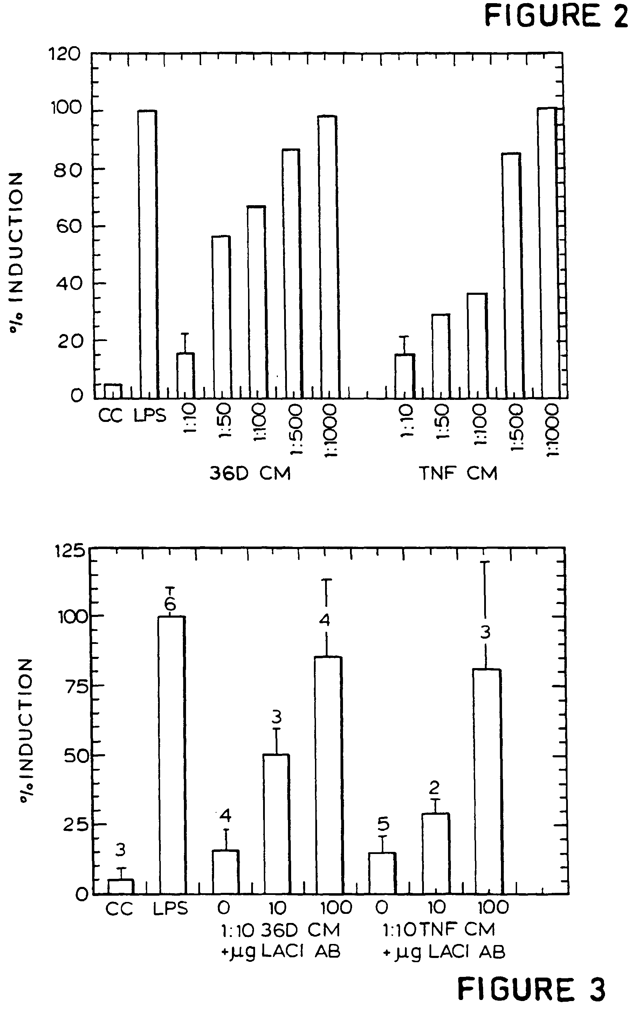

Figure 2 shows the inhibition of tissue factor activity by 36 day conditioned medium (CM) and TNF induced CM.

Figure 3 shows LACI neutralization of CM from endothelial cells.

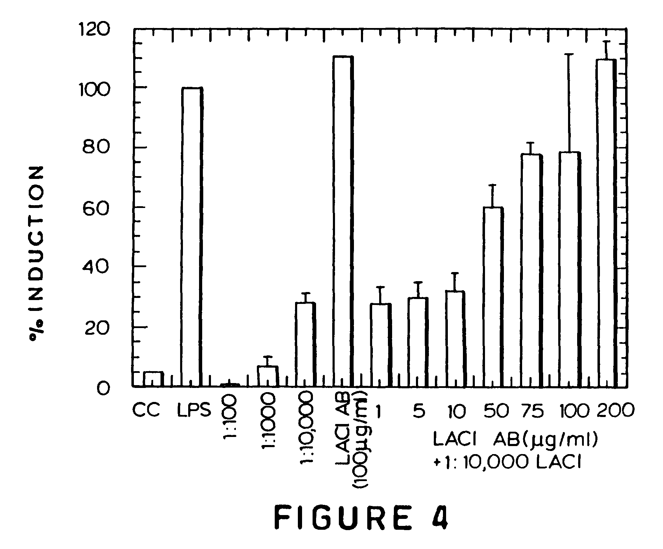

Figure 4 shows antibody neutralization of LACI protein.

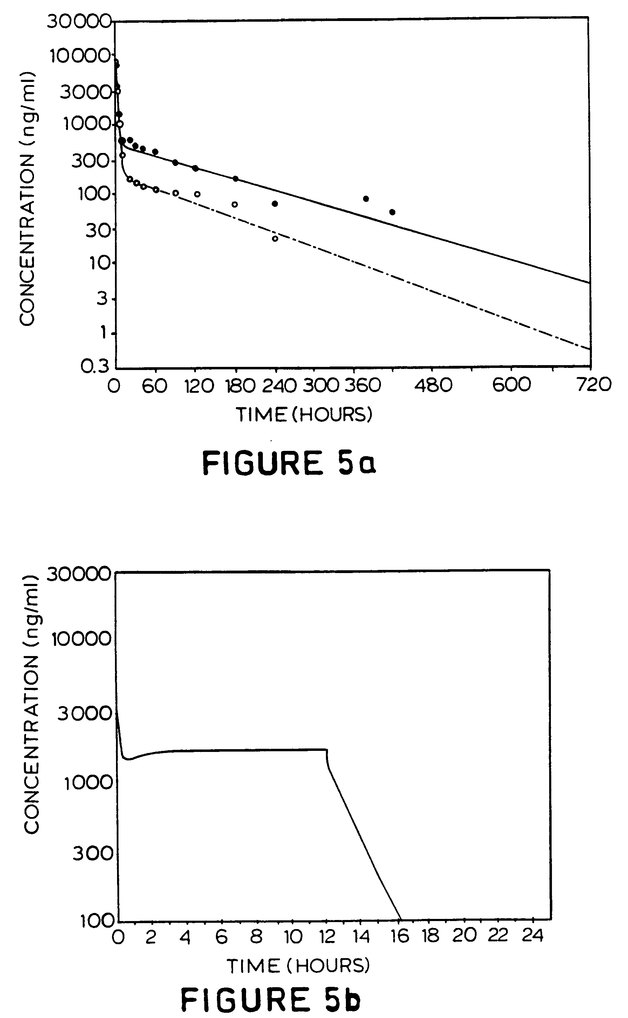

Figures 5a and 5b show pharmacokinetic profile of LACI in baboons. Open circles represent results in the immunoassay and closed circles represent results in the bioassay. For example, 0.5 mg/kg of LACI was given as an I.V. bolus over 30 seconds to two healthy baboons. Blood was sampled from animals at +1 minute, 3, 6, 10, 20, 40, 60, 90, 120, 180, 240 and 420 minutes. LACI levels in plasma were measured using both immunoassay and bioassay (described in text). In Figure 5b, the line represents 0.7 ug/kg + 10 ug/kg/min inf. 12 hr.

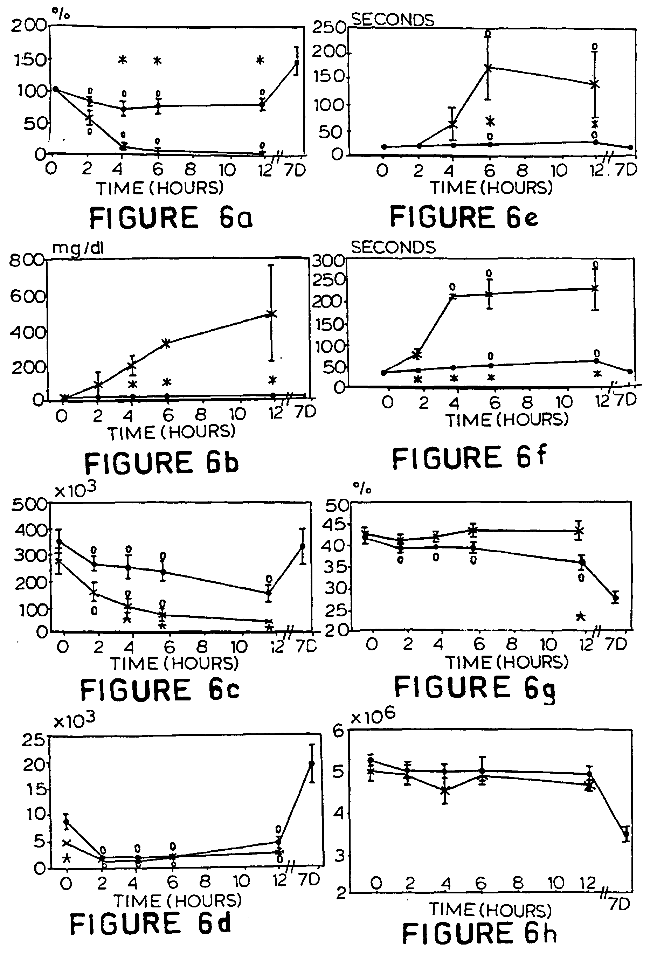

Figures 6a through 6h show the coagulation and hematological response to LACI administration 30 minutes after the start of a two hour lethal bacterial intravenous infusion. Lines with solid circles represent results obtained from treated animals and lines with "Xs" represent results obtained from control animals. A * (star) indicates a statistically significant difference (p< 0.05) between the control and experimental groups and an open circle represents a statistically significant (p< 0.05) difference between times. A Figure 6a shows fibrinogen levels, Figure 6b shows FDP levels, Figure 6c shows platelet levels, Figure 6d shows WBC levels, Figure 6e shows PT levels, Figure 6f shows APTT levels, Figure 6g shows hematocrit levels, and Figure 6h shows RBC levels. For example, anesthetized baboons were challenged with a lethal dose of E. coli ($\sim$5 x $10^{10}$ organisms/kg) intravenously infused over two hours. Thirty minutes after the start of the bacterial infusion five baboons received phosphate buffered saline (PBS; excipient control; *) and the other five received LACI in PBS (●). Blood samples were obtained from the ten baboons before the start of the bacterial infusion, and at 2, 4, 6, and 12 hours after the onset of infusion. Blood samples were assayed for fibrinogen, fibrin degradation products, prothrombin tine, activated partial thromboplastin time, and for hematocrit, platelet, red cell and white cell counts by standard methods. Mean ± standard error of each measurement is plotted against time (hrs.).

Table 1 shows the weight, sex, *E. coli* dose and survival times of control and LACI treated baboons at +30 minutes.

Table 2 shows the clinical chemistry summary of LACI treated and control baboons at +30 minutes.

Table 3 shows IL-6 levels (ng/ml) after LACI administration at +30 minutes.

Table 4 shows the weight, sex, *E. coli* dose and survival times of control and LACI treated baboons at +240 minutes.

Detailed Description of the Invention

LACI

As stated above, the present invention is the discovery that LACI can inhibit the deleterious effects of septic shock. Specifically, LACI inhibits/attenuates the coagulopathies and the inflammatory process associated with acute inflammatory and septic shock. LACI is a serum glycoprotein with a molecular weight of 38,000 Kd. It is also known as a tissue factor inhibitor because it is a natural inhibitor of thromboplastin (tissue factor) induced coagulation (U.S. Patent Nos. 5,110,730 and 5,106,833 describe tissue factor). LACI is a protease inhibitor and has 3 Kunitz domains, two of each are known to interact with factors VII and Xa respectively, while the function of the third domain is unknown. Many of the structural features of LACI can be deduced because of its homology with other well-studied proteases. LACI is not an enzyme, so it probably inhibits its protease target in a stoichiometric manner; namely, one of the domains of LACI inhibits one protease molecule. LACI is defined as including fragments which maintain anti-sepsis activity and hybrid molecules which include LACI. See U.S. Patent No. 5,106,833 for LACI fragments and muteins.

LACI was discovered by Broze et al., 1987, PNAS (USA), 84:1886-1890, and was found to inhibit Factor Xa directly, as well as to inhibit tissue factor activity by formation of an inert factor VIIa/tissue factor (TF)/Factor Xa/Ca++ inhibitor complex. It has the DNA sequence shown in U.S. Patent No. 4,966,852. A schematic diagram of the proposed mechanism for the inhibition of Factor Xa and VIIa/TF complex by LACI is shown in Figure 1.

Coagulation occurs via two pathways; intrinsic and extrinsic. The intrinsic and extrinsic pathways of coagulation consist of several proteases that are activated in a series which, unless inhibited, result in the formation of fibrin clots. LACI acts at two steps in the coagulation cascade pathway both at the Xa and VIIa/TF level as described above. The activation of tissue factor, which LACI inhibits, is a relatively early event in extrinsic pathway. (LACI has also been called Extrinsic Pathway Inhibitor (EPI) and tissue factor pathway inhibitor (TFPI)). LACI inactivates Factor Xa which is a common protease for the extrinsic and intrinsic pathway and is downstream from activation of tissue factor.

The concentration of LACI in normal plasma is 100 ng/ml. A report by Bajaj et al., 1987, J. Clin. Invest., 79:1874-1878, suggests that LACI is synthesized in liver and endothelial cells and is consumed during DIC in patients. Specifically, LACI values in the plasma of 15 healthy volunteers ranged from 72 to 142 U/ml with a mean of 101 U/ml. Interestingly, LACI levels of 10 patients with DIC were $57 \pm 30$ U/ml (p < .001). In contrast, LACI levels of 12 patients with hepatocellular disease were a mean of $107 \pm 33$, i.e., similar to normal. Sandset et al., 1989, J. Internal Med., 225:311-316, monitored LACI plasma levels during a 7-day observation period from patients with pneumonia (n=13), and in stroke patients with infarction (n=9), and haemorrhage (n=9). In pneumonia patients, LACI showed a weak but not significant increase in the recovery period (p=.068). In cerebral haemorrhage patients, LACI levels did not consistently change, while in cerebral infarction patients, an increase in LACI levels was observed from day 1 to day 2 (p < .05). This latter effect was most probably due to release of tissue bound LACI by heparin and thus, was only observed in heparin-treated patients.

Sandset et al., 1989, Haemostasis, 19:189-195, also serially determined LACI levels in 13 patients with postoperative/post-traumatic septicemia. In the survivors (n=8), initial low LACI activity normalized during recovery. In the fatal cases (n=5), a progressive increase in LACI activity (maximal $30 \pm 15\%$) was observed until death. The increase may be explained by a badly damaged endothelium that is releasing the tissue bound LACI into the circulation.

LACI Manufacture

LACI can be made and isolated by several methods. For example, cells that secrete LACI include aged endothelial cells or young endothelial cells which have been treated with TNF for about 3 to 4 days, also hepatocytes or hepatoma cells. LACI can be purified from this cell culture by conventional methods. For example, these methods include the chromatographic methods shown in Pedersen et al., 1990, J. of Biological Chemistry, 265:16786-16793, Novotny et al., 1989, J. of Biological Chemistry, 264:18832-18837, Novotny et al., 1991, Blood, 78:394-400, Wun et al., 1990, J. of Biological Chemistry, 265;16096-16101, and Broze et al., 1987, PNAS (USA), 84:1886-1890. Furthermore, LACI appears in the bloodstream and could be purified from blood, see Pedersen et al., supra. However, that method is not suggested or preferred because of the large quantities of blood that would be required to obtain sufficient quantities of LACI.

LACI may be produced recombinantly as shown in U.S. Patent No. 4,966,852. For example, the cDNA for the protein can be incorporated into a plasmid for expression in prokaryotes or eukaryotes. U.S. Patent No. 4,847,201 provides details for transforming microorganisms with specific DNA sequences and expressing them. There are many other references known to those of ordinary skill in the art which provide details on expression of proteins using microorganisms. Many of those are cited in U.S. Patent No. 4,847,201, such as Maniatas T., et al., 1982, Molecular Cloning, Cold Spring Harbor Press.

The following is an overview about transforming and expressing LACI in microorganisms. LACI DNA sequences must be isolated, and connected to the appropriate control sequences. LACI DNA sequences are shown in U.S. Patent No. 4,966,852 and it can be incorporated into a plasmid, such as pUNC13 or pBR3822, which are commercially available from companies such as Boehringer-Mannheim. Once the LACI DNA is inserted into a vector, it can be cloned into a suitable host. The DNA can be amplified by techniques such as those shown in U.S. Patent No. 4,683,202 to Mullis and U.S. Patent No. 4,683,195 to Mullis et al. (LACI cDNA may be obtained by inducing cells, such as hepatoma cells (such as HepG2 and SKHep) to make LACI mRNA then identifying and isolating the mRNA and reverse transcribing it to obtain cDNA for LACI.) After the expression vector is transformed into a host such as *E. coli* the bacteria may be fermented and the protein expressed. Bacteria are preferred prokaryotic microorganisms and *E. coli* is especially preferred. A preferred microorganism useful in the present invention is *E. coli* K-12, strain MM294 deposited with the ATCC on February 14, 1984, under the provisions of the Budapest Treaty. It has accession number 39607. Alternatively, LACI may be introduced into mammalian cells. These mammalian cells may include CHO, COS, C127, Hep G2, SK Hep, baculovirus, and infected insect cells (see also U.S. Patent No. 4,847,201, referred to above). See also Pedersen et al., 1990, J. of Biological Chemistry, 265:16786-16793. Some specific details about the production of a recombinant protein typically involves the following:

Suitable Hosts, Control Systems and Methods

First, a DNA encoding the mature protein (used here to include all muteins); the preprotein; or a fusion of the LACI protein to an additional sequence which does not destroy its activity or to additional sequence cleaved under controlled conditions (such as treatment with peptidase) to give an active protein, is obtained. If the sequence is uninterrupted by introns it is suitable for expression in any host. If there are introns, expression is obtainable in mammalian or other eucaryotic systems capable of processing them. This sequence should be in excisable and recoverable form. The excised or recovered coding sequence is then placed in operable linkage with suitable control sequences in a replicable expression vector. The vector is used to transform a suitable host and the transformed host cultured under favorable conditions to effect the production of the recombinant LACI.

Genomic or cDNA fragments are obtained and used directly in appropriate hosts. The constructions for expression vectors operable in a variety of hosts are made using appropriate replications and control sequences, as set forth below. Suitable restriction sites can, if not normally available, be added to the ends of the coding sequence so as to provide an excisable gene to insert into these vectors.

The control sequences, expression vectors, and transformation methods are dependent on the type of host cell used to express the gene. Generally, procaryotic, yeast, or mammalian cells are presently useful as hosts. Host systems which are capable of proper post-translational processing are preferred. Accordingly, although procaryotic hosts are in general the most efficient and convenient for the production of recombinant proteins, eucaryotic cells, and, in particular, mammalian cells are preferred for their processing capacity, for example, the ability to form the proper glycosylation patterns. In addition, there is more assurance that the native signal sequence will be recognized by the mammalian host cell, thus making secretion possible, and purification thereby easier.

Control Sequences and Corresponding Hosts

Procaryotes most frequently are represented by various strains of E. coli. However, other microbial strains may also be used, such as bacilli, for example Bacillus subtilis, various species of Pseudomonas, or other bacterial strains. In such procaryotic systems, plasmid vectors which contain replication sites and control sequences derived from a species compatible with the host are used. For example, E. coli is typically transformed using derivatives of pBR322, a plasmid derived from an E. coli species by Boilvar, et al., 1977, Gene, 2:95. pBR322 contains genes for ampicillin and tetracycline resistance, and thus provides additional markers which can be either retained or destroyed in constructing the desired vector. Commonly used procaryotic control sequences are defined herein to include promoters for transcription initiation, optionally with an operator, along with ribosome binding site sequences, which include such commonly used promoters as the beta-lactamase (penicillinase) and lactose (lac) promoter systems (Chang, et al., 1977, Nature, 198:1056) and the tryptophan (trp) promoter system (Goeddel, et al., 1980, Nucleic Acids Res., 8:4057) and the λ derived P_L promoter and N-gene ribosome binding site (Shimatake, et al., 1981, Nature, 292:128), which has been made useful as a portable control cassette, as set forth in U.S. Patent No. 4,711,845, issued December 8, 1987. However, any available promoter system compatible with procaryotes can be used.

In addition to bacteria, eucaryotic microbes, such as yeast, may also be used as hosts. Laboratory strains of Saccharomyces cerevisiae, Baker's yeast, are most used although a number of other strains are commonly available. Examples of plasmid vectors suitable for yeast expression are shown in Broach, J.R., 1983, Meth. Enz., 101:307; Stinchcomb et al., 1979, Nature, 282:39; and Tschempe et al., 1980, Gene, 10:157 and Clarke, L., et al., 1983, Meth. Enz., 101:300. Control sequences for yeast vectors include promoters for the synthesis of glycolytic enzymes (Hess, et al., 1968, J. Adv. Enzyme Reg., 7:149; Holland, et al., 1978, Biochemistry, 17:4900). Additional promoters known in the art include the promoter for 3-phosphoglycerate kinase (Hitzeman, et al., 1980, J. Biol. Chem., 255:2073), and those for other glycolytic enzymes, such as glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other promoters, which have the additional advantage of transcription controlled by growth conditions, are the promoter regions for alcohol dehydrogenase 2, isocytochrome C, acid phosphatase, degradative enzymes associated with nitrogen metabolism, and enzymes responsible for maltose and galactose utilization (Holland, supra). It is also believed that terminator sequences are desirable at the 3' end of the coding sequences. Such terminators are found in the 3' untranslated region following the coding sequences in yeast-derived genes. Many of the vectors illustrated contain control sequences derived from the enolase gene containing plasmid peno46 (Holland, M.J. et al., 1981, J. Biol. Chem., 256:1385) or the LEU2 gene obtained from YEp13 (Broach, J. et al., 1978, Gene, 8:121), however, any vector containing a yeast compatible promoter, origin of replication and other control sequences is suitable.

It is also, of course, possible to express genes encoding polypeptides in eucaryotic host cell cultures derived from multicellular organisms. See, for example, Tissue Culture, 1973, Cruz and Patterson, eds., Academic Press. Useful host cell lines include murine myelomas N51, VERO, HeLa cells, Chinese hamster ovary (CHO) cells, COS, C127, Hep G2, SK Hep, baculovirus, and infected insect cells. Expression vectors for such cells ordinarily include promoters and control sequences compatible with mammalian cells such as, for example, the commonly used early and later promoters from Simian Virus 40 (SV4O) (Fiers, et al., 1978, Nature, 273:113), or other viral promoters such as those derived from polyoma, Adenovirus 2, bovine papilloma virus, or avian sarcoma viruses, or immunoglobulin promoters and heat shock promoters. General aspects of mammalian cell host system transformations have been described by Axel, U.S. Patent No. 4,399,216, issued August 16, 1983. It now appears also that "enhancer" regions are important in optimizing expression: these are, generally, sequences found upstream of the promoter region. Origins of replication may be obtained, if needed, from viral sources. However, integration into the chromosome is a common mechanism for DNA replication in eucaryotes. Plant cells are also now available as hosts, and control sequences compatible with plant cells such as the nopaline synthase promoter and polyadenylation signal sequences (Depicker. A., et al., 1982, J. Mol. Appl. Gen., 1:561) are available. Methods and vectors for transformation of plant cells have been disclosed in PCT Publication No. WO 85/04899, published November 7, 1985.

Host strains useful in cloning and expression herein are as follows:

For cloning and sequencing, and for expression of construction under control of most bacterial promoters, E. coli strain MM294 obtained from E. coli Genetic Stock Center GCSC #6135. For expression under control of the $P_LN_{RBS}$ promoter, E. coli strain K12 MC1000 lambda lysogen, $N_7N_{53}$cl857 SusP80, a strain deposited with the American Type Culture Collection (ATCC 39531), may be used. E. coli DG116, which was deposited with the ATCC (Accession No. 53606) on April 7, 1987, may also be used.

For M13 phage recombinants, E. coli strains susceptible to phage infection, such as E. coli K12 strain DG98, can be employed. The DG98 strain has been deposited with the ATCC (ATCC 39768) on July 13, 1984.

Mammalian expression can be accomplished in COS-A2 cells, COS-7, CV-1, murine myelomas N51, VERO, HeLa cells, Chinese hamster ovary (CHO) cells, COS, C127, Hep G2, SK Hep, baculovirus, and infected insect cells. Insect cell-based expression can be in Spodoptera frugiperda.

Transformations

Depending on the host cell used, transformation is done using standard techniques appropriate to such cells. The calcium treatment employing calcium chloride, as described by Cohen, S.N., 1972, PNAS (USA), 69:2110, is used for procaryotes or other cells which contain substantial cell wall barriers. Infection with Agrobacterium tumefaciens (Shaw, C.H. et al., 1983, Gene, 23:315) is used for certain plant cells. For mammalian cells without such cell walls, the calcium phosphate precipitation method of Graham and van der Eb, 1987, Virology, 52:546 is preferred. Transformations into yeast are carried out according to the method of Van Solingen, P. et al., 1977, J. Bact., 130:946 and Hsiao, C.L. et al., 1979, PNAS (USA), 76:3829.

Probing mRNA by Northern Blot: Probe of cDNA or Genomic Libraries

RNA is fractionated for Northern blot by agarose slab gel electrophoresis under fully denaturing conditions using formaldehyde, Maniatas, T., et al., 1982, Molecular Cloning, Cold Spring Harbor Press, pp. 202-203, or 10 mM methyl mercury (CH$_3$HgOH) (Bailey, J.M., et al., 1976, Anal. Biochem., 70:75-85; Shegal, P.B. et al., 1980, Nature, 288:95-97) as the denaturant. For methyl mercury gels, 1.5% gels are prepared by melting agarose in running buffer (100 mM boric acid, 6 mM sodium borate, 10 mM sodium sulfate, 1 mM EDTA, pH 8.2), cooling to 60°C and adding 1/100 volume of 1 M CH$_3$HgOH. The RNA is dissolved in 0.5 x running buffer and denatured by incubation in 10 mM methyl mercury for 10 minutes at room temperature. Glycerol (20%) and bromophenol blue (0.05%) are added for loading the samples. Samples are electrophoresed for 500-600 volt-hr with recirculation of the buffer. After electrophoresis, the gel is washed for 40 minutes in 10 mM 2-mercaptoethanol to detoxify the methyl mercury, and Northern blots prepared by transferring the RNA from the gel to a membrane filter.

cDNA or genomic libraries are screened using the colony or plaque hybridization procedure. Bacterial colonies, or the plaques for phage, are lifted onto duplicate nitrocellulose filter papers (S&S type BA-85). The plaques or colonies are lysed and DNA is fixed to the filter by sequential treatment for 5 minutes with 500 mM NaOH, 1.5 M NaCl. The filters are washed twice for 5 minutes each time with 5 x standard saline citrate (SSC) and are air dried and baked at 80°C for 2 hours.

The gels for Northern blot or the duplicate filters for cDNA or genomic screening are prehybridized at 25° to 42°C for 6 to 8 hours with 10 ml per filter of DNA hybridization buffer without probe (0-50% formamide, 5-6 x SSC, pH 7.0, 5 x Denhardt's solution (polyvinylpyrrolidone, plus Ficoll and bovine serum albumin: 1 x = 0.02% of each), 20-50 mM sodium phosphate buffer at pH 7.0, 0.2% sodium dodecyl sulfate (SDS), 20 μg/ml poly U (when probing cDNA), and 50 μg/ml denatured salmon sperm DNA). The samples are then hybridized by incubation at the appropriate temperature for about 24-36 hours using the hybridization buffer containing kinased probe (for oligomers). Longer cDNA or genomic fragment probes were labelled by nick translation or by primer extension.

The conditions of both prehybridization and hybridization depend on the stringency desired, and vary, for example, with probe length. Typical conditions for relatively long (e.g., more than 30-50 nucleotide) probes employ a temperature of 42° to 55°C and hybridization buffer containing about 20%-50% formamide. For the lower stringencies needed for oligomeric probes of about 15 nucleotides, lower temperatures of about 25°-42°C. and lower formamide concentrations (0%-20%) are employed. For longer probes, the filters may be washed, for example, four times for 30 minutes, each time at 40° - 55°C with 2 x SSC, 0.2% SDS and 50 mM sodium phosphate buffer at pH 7, then washed twice with 0.2 x SSC and 0.2% SDS, air dried, and are autoradiographed at -70°C for 2 to 3 days. Washing conditions are somewhat less harsh for shorter probes.

Vector Construction

Construction of suitable vectors containing the desired coding and control sequences employs standard ligation and restriction techniques which are well understood in the an. Isolated plasmids, DNA sequences, or synthesized oligonucleotides are cleaved, tailored, and religated in the form desired.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 μg of plasmid or DNA sequence is cleaved by 1 unit of enzyme in about 20 μl of buffer solution; in the examples herein, typically, an excess of restriction enzyme is used to insure complete digestion of the DNA substrate. Incubation times of about 1 hour to 2 hours at about 37°C are workable, although variations can be tolerated. After each incubation, protein is removed by extraction with phenol/chloroform, and may be followed by ether extraction, and the nucleic acid recovered from aqueous fractions by precipitation with ethanol. If desired, size separation of the cleaved fragments may be performed by polyacrylamide gel or agarose gel electrophoresis using standard techniques. A general description of size separations is found in Methods of Enzymology, 1980, 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 minutes at 20° to 25°C in 50 mM dithiothreitol (DTT) and 5-10 μM dNTPs. The Klenow fragment fills in at 5' sticky ends but chews back protruding 3' single strands, even though the four dNTPs are present. If desired, selective repair can be performed by supplying only one of the, or selected, dNTPs within the limitations dictated by the nature of the sticky ends. After treatment with Klenow, the mixture is extracted with phenol/chloroform and ethanol precipitated. Treatment under appropriate conditions with S1 nuclease results in hydrolysis of any single-stranded portion.

Synthetic oligonucleotides may be prepared by the triester method of Matteucci et al., 1981, J. Am. Chem. Soc., 103:3185-3191, or using automated synthesis methods. Kinasing of single strands prior to annealing or for labelling is achieved using an excess, e.g., approximately 10 units of polynucleotide kinase to 1 nmole substrate in the presence of 50 mM Tris, pH 7.6, 10 mM $MgCl_2$, 5 mM DTT, 1-2 mM ATP. If kinasing is for labelling of probe, the ATP will contain high specific activity 32YP.

Ligations are performed in 15-30 $\mu$l volumes under the following standard conditions and temperatures: 20 mM Tris-Cl pH 7.5, 10 mM $MgCl_2$, 10 mM DTT, 33 $\mu$g/ml bovine serum albumin (BSA), 10 mM-50 mM NaCl, and either 40 $\mu$M ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 $\mu$g/ml total DNA concentrations (5-100 nM total end concentration). Intermolecular blunt end ligations (usually employing a 10-30 fold molar excess of linkers) are performed at 1 $\mu$M total ends concentration.

In the vector construction employing "vector fragments", the vector fragment is commonly treated with bacterial alkaline phosphatase (BAP) in order to remove the 5' phosphate and prevent religation of the vector. BAP digestions are conducted at pH 8 in approximately 150 mM Tris, in the presence of $Na^{2+}$ and $Mg^{2+}$ using about 1 unit of BAP per $\mu$g of vector at 60°C for about 1 hour. In order to recover the nucleic acid fragments, the preparation is extracted with phenol/chloroform and ethanol precipitated. Alternatively, religation can be prevented in vectors which have been double digested by additional restriction enzyme digestion of the unwanted fragments.

## Modification of DNA Sequences

For portions of vectors derived from cDNA or genomic DNA which require sequence modifications, site specific primer directed mutagenesis is used. This technique is now standard in the art, and is conducted using a primer synthetic oligonucleotide complementary to a single stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form: 50% will have the original sequence. The plaques are hybridized with kinased synthetic primer at a temperature which permits hybridization of an exact match, but at which the mismatches with the original strand are sufficient to prevent hybridization. Plaques which hybridize with the probe are then picked, cultured, and the DNA recovered.

## Verification of Construction

Correct ligations for plasmid construction could be confirmed by first transforming *E. coli* strain MM294, or other suitable host, with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D.B. et al., 1969, PNAS (USA), 62:1159, optionally following chloramphenicol amplification (Clewell, D.B., 1972, J. Bacteriol, 110:667). The isolated DNA is analyzed by restriction and/or sequenced by the dideoxy method of Sanger, F., et al., 1977, PNAS (USA), 74:5463 as further described by Messing et al., 1981, Nucleic Acids Res., 9:309, or by the method of Maxam et al., 1980, Methods in Enzymology, 65:499.

## Purification of LACI

For purification of mammalian cell expressed LACI, the following methods may be used: sequential application of heparin-Sepharose, MonoQ, MonoS, and reverse phase HPLC chromatography. See Pedersen et al., supra, Novotny et al., 1989, J. of Biological Chemistry, 264:18832-18837, Novotny et al., 1991, Blood, 78:394-400, Wun et al., 1990, J. of Biological Chemistry, 265;16096-16101, and Broze et al., 1987, PNAS (USA), 84: 1886-1890. These references describe various methods for purifying mammalian produced LACI.

Additionally, LACI may be produced in bacteria, such as *E. coli*, and subsequently purified. Generally, the procedures shown in U.S. Patent Nos. 4,511,502; 4,620,948; 4,929,700; 4,530,787; 4,569,790; 4,572,798; and 4,748,234 can be employed. These patents are hereby incorporated by reference in their entireties. Typically, the heterologous protein (i.e. LACI) is produced in a refractile body within the bacteria. To recover and purify the protein, the cells are lysed and the refractile bodies are centrifuged to separate them from the cellular debris (see U.S. Patent No. 4,748,234 for lowering the ionic strength of the medium to simplify the purification). Thereafter, the refractile bodies containing the LACI are denatured, at least once (typically in reducing environment), and the protein is oxidized and refolded in an appropriate buffer solution for an appropriate length of time. LACI has a significant number of cysteine residues and the procedure shown in U.S. Patent No. 4,929,700 should be relevant because CSF-1 also contains a significant number of cysteine residues. LACI may be purified from the buffer solution by various chromatographic methods, such as those mentioned above for the mammalian cell derived LACI. Additionally, the methods shown in U.S. Patent No. 4,929,700 may be employed.

## Formation and Administration

LACI is administered at a concentration that is therapeutically effective to treat and prevent sepsis, acute or chronic inflammation, and other diseases in which cytokines up regulate tissue factor. To accomplish this goal, LACI is preferably administered intravenously. Methods to accomplish this administration are known to those of ordinary skill in the art.

Before administration to patients, formulants may be added to LACI. A liquid formulation is preferred. In the example below, LACI was formulated in 150 mM NaCl and 20 mM NaPO$_4$ at pH 7.2. However, LACI may be formulated at different concentrations or using different formulants. For example, these formulants may include oils, polymers, vitamins, carbohydrates, amino acids, salts, buffers, albumin, surfactants, or bulking agents. Preferably carbohydrates include sugar or sugar alcohols such as mono, di, or polysaccharides, or water soluble glucans. The saccharides or glucans can include fructose, dextrose, lactose, glucose, mannose, sorbose, xylose, maltose, sucrose, dextran, pullulan, dextrin, alpha and beta cyclodextrin, soluble starch, hydroxethyl starch and carboxymethylcelloluose, or mixtures thereof. Sucrose is most preferred. Sugar alcohol is defined as a C$_4$ to C$_8$ hydrocarbon having an -OH group and includes galactitol, inositol, mannitol, xylitol, sorbitol, glycerol, and arabitol. Mannitol is most preferred. These sugars or sugar alcohols mentioned above may be used individually or in combination. There is no fixed limit to amount used as long as the sugar or sugar alcohol is soluble in the aqueous preparation. Preferably, the sugar or sugar alcohol concentration is between 1.0 w/v% and 7.0 w/v%, more preferable between 2.0 and 6.0 w/v%. Preferably amino acids include levorotary (L) forms of carnitine, arginine, and betaine; however, other amino acids may be added. Preferred polymers include polyvinylpyrroildone (PVP) with an avenge molecular weight between 2,000 and 3,000, or polyethylene glycol (PEG) with an average molecular weight between 3,000 and 5,000. It is also preferred to use a buffer in the composition to minimize pH changes in the solution before lyophilization or after reconstitution. Most any physiological buffer may be used, but citrate, phosphate, succinate, and glutamate buffers or mixtures thereof are preferred. Preferably, the concentration is from 0.01 to 0.3 molar. Surfactants that can be added to the formulation are shown in EP Nos. 270,799 and 268,110.

Additionally, LACI can be chemically modified by covalent conjugation to a polymer to increase its circulating half-life, for example. Preferred polymers, and methods to attach them to peptides, are shown in U.S. Patent Nos. 4,766,106, 4,179,337, 4,495,285, and 4,609,546 which are all hereby incorporated by reference in their entireties. Preferred polymers are polyoxyethylated polyols and polyethylene glycol (PEG). PEG is soluble in water at room temperature and has the general formula: R(O-CH$_2$-CH$_2$)$_n$O-R where R can be hydrogen, or a protective group such as an alkyl or alkanol group. Preferably, the protective group has between 1 and 8 carbons, more preferably it is methyl. The symbol n is a positive integer, preferably between 1 and 1,000, more preferably between 2 and 500. The PEG has a preferred average molecular weight between 1000 and 40,000, more preferably between 2000 and 20,000, most preferably between 3,000 and 12,000. Preferably, PEG has at least one hydroxy group, more preferably it is a terminal hydroxy group. It is this hydroxy group which is preferably activated to react with a free amino group on the inhibitor. However, it will be understood that the type and amount of the reactive groups may be varied to achieve a covalently conjugated PEG/IL-2 of the present invention.

Water soluble polyoxyethylated polyols are also useful in the present invention. They include polyoxyethylated sorbitol, polyoxyethylated glucose, polyoxyethylated glycerol (POG), etc. POG is preferred. One reason is because the glycerol backbone of polyoxyethylated glycerol is the same backbone occurring naturally in, for example, animals and humans in mono-, di-, triglycerides. Therefore, this branching would not necessarily be seen as a foreign agent in the body. The POG has a preferred molecular weight in the same range as PEG. The structure for POG is shown in Knauf et al., 1988, J. Bio. Chem. 263:15064-15070, and a discussion of POG/IL-2 conjugates is found in U.S. Patent No. 4,766,106.

After the liquid pharmaceutical composition is prepared, it is preferably lyophilized to prevent degradation and to preserve sterility. Methods for lyophilizing liquid compositions are known to those of ordinary skill in the art. Just prior to use, the composition may be reconstituted with a sterile diluent (Ringer's solution, distilled water, or sterile saline, for example) which may include additional ingredients. Upon reconstitution, the composition is preferably administered to subjects using those methods that are known to those skilled in the art.

## Administration to Affected Individuals

As stated above, LACI is useful to therapeutically or prophylactically treat human patients with sepsis or septic shock. Generally, people having sepsis are characterized by high fever (>38.5°C) or hypothermia (<35.5°C), low blood pressure, tachypnea (> than 20 breaths/minute), tachycardia (> than 100 beats/minute), leukocytosis (> 15,000 cells/mm$^3$) and thrombocytopenia (< than 100,000 platelets/mm$^3$) in association with bacteremia. LACI is to be administered as soon as a patient is suspected of being septic; presenting themselves with a greater than or equal to 20% drop in fibrinogen or appearance of fibrin split products, a rise in the patient's temperature and the diagnosis of leukopenia, thrombocytopenia and hypotension associated with sepsis. As also stated above, the preferred route is by intravenous administration. Generally, LACI is given at a dose between 1 µg/kg and 20 mg/kg, more preferably between 20 µg/kg and 10 mg/kg, most preferably between 1 and 7 mg/kg. Preferably, it is given as a bolus dose, to increase circulating levels by 10-20 fold and for 4-6 hours after the bolus dose. Continuous infusion may also be used after the bolus dose. If so, LACI may be infused at a dose between 5 and 20 µg/kg/minute, more preferably between 7 and 15 µg/kg/minute.

LACI may be given in combination with other agents which would be effective to treat sepsis. For example, the following may be administered in combination with LACI: antibiotics that can treat the underlying bacterial infection; monoclonal antibodies that are directed against bacterial cell wall components; receptors that can complex with cytokines that are involved in the sepsis pathway; and generally any agent or protein that can interact with cytokines or complement proteins in the sepsis pathway to reduce their effects and to attenuate sepsis or septic shock.

Antibiotics that are useful in the present invention include those in the general category of: beta-lactim rings (penicillin), amino sugars in glycosidic linkage (amino glycosides), macrocyclic lactone rings (macrolides), polycyclic derivatives of napthacenecarboxamide (tetracyclines), nitrobenzene derivatives of dichloroacetic acid, peptides (bacitracin, gramicidin, and polymyxin), large rings with a conjugated double bond system (polyenes), sulfa drugs derived from sulfanilamide (sulfonamides), 5-nitro-2-furanyl groups (nitrofurans), quinolone carboxylic acids (nalidixic acid), and many others. Other antibiotics and more versions of the above specific antibiotics may be found in Encyclopedia of Chemical Technology, 3rd Edition, Kirk-Othymer (ed.), Vol. 2, pages 782-1036 (1978) and Vol. 3, pages 1-78, Zinsser, MicroBiology, 17th Edition W. Jokilk et al. (Eds.) pages 235-277 (1980), or Dorland's Illustrated Medical Dictionary, 27th Edition, W.B. Saunders Company (1988).

Monoclonal antibodies that may be administered along with LACI include those found in PCT WO 88/03211, to Larrick et al., entitled Gram-Negative Bacterial Endotoxin Blocking Monoclonal Antibodies. The application discloses specific monoclonal antibodies that are useful to treat sepsis and which bind to various antigens on the E. coli bacterial cell wall. A specifically preferred monoclonal antibody is that which is produced by hybridoma ATCC No. HB9431.

Other agents which may be combined with LACI include monoclonal antibodies directed to cytokines involved in the sepsis pathway, such as those monoclonal antibodies directed to IL-6 or M-CSF, see PCT WO91/08774, published June 27, 1991 to Creasey et al. and monoclonal antibodies directed to TNF, see Cerami et al., U. S. Patent No. 4,603,106. Inhibitors of protein that cleave the mature TNF prohormone from the cell in which it was produced, see PCT WO91/02540, published March 7, 1991 to Kriegler et al.. Antagonists of IL-1, such as shown in PCT WO91/17249, published November 14, 1991 to Haskill et al., Inhibitors of IL-6 cytokine expression such as inhibin, such as shown in PCT WO91/12334, published August 22, 1991 to Warren et al., and receptor based inhibitors of various cytokine such as IL-1. Antibodies to complement or protein inhibitors of complement, such as $CR_I$, DAF, and MCP may also be employed.

Generally, LACI may be useful for those diseases that occur due to the up-regulation of tissue factor brought on by TNF, IL-1 or other cytokines. For example, in the examples below, LACI administration is shown to lower the IL-6 concentration. Since IL-6 is one factor that is involved in acute or chronic inflammation, LACI administration is useful for treating inflammation. Typical inflammatory conditions that can be treated by LACI include: arthritis, septic shock, reperfusion injury, inflammatory bowel disease, acute respiratory disease, trauma, and burn.

In treating chronic or acute inflammation, LACI may be administered in the same fashion and at the same dose as the anti-sepsis method.

The present invention will now be illustrated by reference to the following examples which set forth particularly advantageous embodiments.

Example 1

Production of LACI

A. Aged Cells

Human umbilical vein endothelial cells (HuVec) were plated and maintained in a standard tissue culture medium. They were aged for 32-36 days, fed twice a week with fresh medium, and the medium supernatant was removed after 32 days (called conditioned medium or CM). The CM contained LACI.

B. Induced Cells

The same HuVec cells were plated and maintained in a tissue culture medium for 24-48 hours and then they were contacted with various concentrations of tumor necrosis factor (TNF) for 3-4 days. The medium containing LACI was removed and is called TNF CM.

## Example 2

LACI Inhibition of Sepsis

The following assay was devised to measure the inhibition of sepsis by LACI. HuVec cells were plated and incubated for 48 hours. Bacterial lipopolysaccharide (LPS) was added as an inducer of sepsis. The addition of LPS was the best way to stimulate a sepsis-like response which was broader than simple coagulation. When the inducer was added, a test sample was added to examine its effect on the LPS effect on the endothelial cells. The sample that was tested contained LACI. The cells were incubated between 4 and 5 hours and then chromozyme was added. The chromozyme contains Factors II, VII, IX, and X. This first method measured the inhibition of tissue factor induction and inhibition of activity. In an alternative of the present assay, which measures inhibition of tissue factor activity, the sample was added together with the chromozyme and then incubated for 45 minutes. LACI inhibitory activity was measured by reading optical density (due to color changes) in a spectrophotometer at $A_{405}$.

Aged and TNF induced condition medium was prepared as in Example 1. Figure 2 displays a dose dependent inhibition of tissue factor activity by a substance contained in the respective media shown in the figure. The nature of the substance was identified by the following experiment which involved inhibition of tissue factor activity determined as follows: HuVec cells were prepared for the assay. One cell sample was left untreated as a control. Another cell sample was induced with LPS without the addition of any potential inhibitor. Subsequently, six classes of samples were run using aged and TNF condition medium containing LACI with 0, 10, and 100 mg of LACI antibody. Figure 3 shows the result of this experiment. For example, (Lane 1 starting from the left) was the control and very little tissue factor activity was detected. Lane 2 shows 100% of tissue factor activity and induction by addition of LPS. Lanes 3, 4, and 5 show linearly increasing amounts of activity (and thus induction) depending on the amount of anti-LACI antibody. For example, the 0 concentration (Lane 3) showed that very little tissue factor activity was detectable, suggesting lack of tissue factor induction. This indicated that LACI inhibited the activity of the tissue factor induced by LPS. Lanes 4 and 5 show a similar result, however, the amount of tissue factor activity/induction increased as larger amounts of LACI were neutralized by the anti-LACI antibody. Lanes 6, 7, and 8 (with TNF conditioned medium) also display a nearly identical magnitude of inhibition of tissue factor activity as that shown for Lanes 3, 4, and 5. To confirm the identity of the substance in the conditioned media, we used various concentrations of highly purified LACI in the absence or presence of neutralizing antibodies. The results match the findings utilizing aged and TNF induced conditioned media. See Figure 4.

These data indicate that LACI will inhibit the effects of LPS on HuVec cells in a concentration dependent manner and this effect may be reversed upon the addition of various concentrations of neutralizing antibodies to LACI. Furthermore, this model proves that LACI can be used to treat sepsis, and its effects were not simply restricted to its anticoagulant properties.

## Example 3

Treatment of Human Patients Using LACI

Human patients which are affected by sepsis may be therapeutically treated by using LACI. When the patient presents themselves with increased temperature, drop in blood pressure, a decrease in white cell count, and a drop $\geq$ 20% in fibrinogen, LACI is administered intravenously as a bolus dose of 3-10 mg/kg and as an infusion of 10-20 μg/kg/min for 3-4 hours. Alternatively, LACI may be administered at a continuous rate of approximately 10 mg/kg/min for 3 days or for 4 hours daily for 3-4 days. Antimicrobial therapy or broad spectrum antibiotics are administered to the patient along with the LACI.

LACI is given prophylactically in the same manner.

## Example 4

In this experiment, highly purified recombinant LACI (6 mg/kg) was administered either thirty minutes or four hours after the start of a lethal intravenous *E. coli* infusion in baboons. Early post treatment of LACI resulted in a) permanent 7 day survivors (5/5) with significant improvement in quality of life, while the mean survival time for the controls (5/5) was 39.9 hrs. (no survivors); b) significant attenuations of the coagulation response and various measures of cell injury, with significant reductions in pathology observed in *E. coli* sepsis target organs including kidneys, adrenals and lungs. LACI administration did not affect the drop in mean systemic arterial pressure, the increases in respiration and heart rate or temperature changes associated with the bacterial infusion. LACI treated *E. coli* infected baboons had twenty fold lower IL-6 levels than their phosphate buffered saline treated controls. In contrast to the earlier 30 minute treatment, the administration of LACI at four hours i.e., 240 minutes, after the start of bacterial infusion resulted in prolongation of survival time, with forty percent improvement in survival rate (two survivors) and some attenuation of the coagulopathic response, especially in animals in which fibrinogen levels were above 10% of normal at the time of LACI administration.

Recombinant Tissue Factor Pathway Inhibitor

LACI was expressed in the human hepatoma cell line SK Hep as described in Wun et al. 1992, Thrombosis Haemost., 68:54-59. Detection of Bacterial Endotoxins with the Limulus Amebocyte Lysate Test, Alan R. Liss, Inc., NY. The material was purified by standard techniques to provide > 95% pure preparations. LACI was formulated in 150 mM NaCl and 20 mM $NaPO_4$ (pH 7.2), which served as the excipient control. Final protein concentration in a LACI sample ranged from 2.3 - 3.7 mg/ml, determined by amino acid composition; endotoxin levels ranged from 8 to 27 endotoxin units per 15 milligrams of protein. LACI lots were monitored for biological activity using a tissue factor inhibition assay (Boze et al., Blood (1988) 71:335-343).

Baboons

Male and female Papio anubis baboons (7.6 ± 2.4 kg) from the Charles River Primate Center (Wilmington, MA) were quarantined for a minimum of 30 days in the University of Oklahoma Health Sciences Center Animal Resource Facility (Oklahoma City, OK). Animals were free of infections or parasites with hematocrits ≥ 36%.

Bacteria

Escherichia coli 086:K61H organisms (ATCC 33985; Rockville, MD) were isolated from a stool specimen at Children's Memorial Hospital, Oklahoma City. They were stored in the lyophilized state at 4°C after growth in tryptic soybean agar and reconstituted and characterized as described in Hinshaw a al., J. Trauma (1982) 23:361-365.

Assays

Endotoxin Measurement

Endotoxin levels in LACI preparations and the excipient buffer were monitored by the limulus amebocyte lysate test (Wun et al., Thromb. Haemost. (1992) 68:54-59). LPS from E. coli (B5505; Mallinckrodt, St. Louis, MO) were included as a standard. The detection limit of the assay was 10 endotoxin units (E.U.)/ml.

TNF ELISA

Baboon TNF levels in plasma were measured using an ELISA developed for detecting human TNF (Creasey et al., Circ. Shock (1991) 33:84-91): a purified monoclonal anti-TNF antibody (24510E11) was bound to microtiter plate wells (Dynatech Immunolon I, Fisher). Unoccupied binding sites on the plastic were then blocked with bovine serum albumin (BSA). Aliquots of standard concentrations of purified recombinant human TNF or baboon plasma samples were incubated in duplicate. ELISA wells were exposed to horseradish peroxidase (HRP)-conjugated affinity packed polyclonal rabbit antibody to recombinant human TNF followed by 0-phenylenediamine substrate as chromogen. Wells were rinsed repeatedly with phosphate-buffered saline solution (PBS, Ph 7.5) between successive incubations. Optical density (OD) was read on an automated dual-wavelength plate reader at 490 nm (Bio-Tek Instruments). The detection limit for baboon TNF in this assay was 0.5 ng/ml.

IL-6 Bioassay

IL-6 bioactivity was quantified in baboon plasma using the IL-6-dependent murine hybridoma cell line B9, using IL-6 commercially available from Amgen, Inc. (Thousand Oaks, CA), as the assay standard (Creasey et al., supra). The detection limits of this assay were 10 pg/ml.

LACI Levels

A competitive fluorescent immunoassay for LACI was used as previously described in Novotny et al., Blood (1991) 78:394-400: a rabbit anti-LACI IgG was used to capture LACI in the sample to be tested and FITC-LACI (HepG2) was added to quantitate the number of anti-LACI binding sites remaining. Standard curves were constructed using dilutions of pooled human plasma (George King Biomedical, Overland Park, KS) or of pure HepG2 LACI.

The LACI functional assay (tissue factor-inhibition assay) is a three-stage clotting assay. Briefly, in the first stage, the sample to be tested is incubated with crude brain tissue factor, factor X, factor VII, and calcium. After 30 minutes of incubation, additional factor X is added and 1 minute later factor X-deficient plasma is added and time to clot is measured in a fibrometer. Residual factor VII(a)/tissue factor activity in the second stage of the assay is inversely proportional to the LACI concentration in the test sample. Thus, prolongation of the clotting time reflects higher LACI activity. Standard curves were constructed using dilutions of pure HepG2 LACI.

Pharmacokinetic Analysis

The data for each baboon ($\mu$g LACI/ml plasma at various sample times) were fit to a two-compartment model. The model parameters were determined by nonlinear least squares curve-fitting procedures using the PKDAAS data analysis system (developed for the VAX computer at Chiron Corporation deposited at the U.S. Copyright Office as registration No. TXU 416-977). Corrected concentrations at each time, C(t), were weighted as the reciprocals of each concentration squared. The weighted values were then fitted to individual subjects' curves using the following biexponential equation:

$$C(t)=(DOSE/VC)^*[(1-B)^*2^{-t/\alpha}+B^*2^{-t/\beta}],$$

where t is time and VC, B, $\alpha$, and $\beta$ are model parameters. The sum of the coefficients was normalized to 1.0. The systemic clearance (CL) was then calculated from:

$$CL=VC/MRT, \text{ where}$$

$$MRT=[(1-B)^*\alpha+B^*\beta]\ln(2).$$

Statistical Analysis

Data were analyzed with the students' t-test to determine significant differences ($p < 0.05$) in means between groups at given times. The analysis of variance (ANOVA) and the multicomparison Duncan's test were used to determine significant differences between means at time 0 and subsequent times within groups. The Fisher's exact test was used to determine significant differences between groups with respect to survival rates.

Pharmacokinetic Studies

To establish the appropriate LACI dosage for the *E. coli* septic shock model, we performed a pharmacokinetic study in three healthy baboons. Figure 5 shows that administered as a bolus at 0.5 mg/kg, LACI exhibited a two phase half life; an alpha phase of approximately two minutes and a beta phase of about two hours. These data were then modeled as described above to identify the necessary LACI dosage to achieve a circulating LACI serum concentration of 2 $\mu$g/ml, which was arbitrarily defined as the desired LACI blood concentration since it has been reported that endogenous levels of LACI in primates is approximately 0.1 $\mu$g/ml (Novotny et al., J. Biol. Chem. (1989) 264:18832-18837). Thus to achieve a 20-fold increase in LACI serum concentrations in the baboons, we administered LACI at a loading dose of 700 $\mu$g/kg and a maintenance dose of 10 $\mu$g/kg/mm (i.e. a total dose of 6,000 $\mu$g/kg) started simultaneously, 30 minutes after the start of the *E. coli* infusion.

Experimental and Infusion Procedures

Each baboon was immobilized with ketamine hydrochloride, 14 mg/kg intramuscularly on the morning of the study and slowly anesthetized with sodium pentobarbital (~9 mg/kg) via a percutaneous catheter positioned in the cephalic vein as described in Hinshaw et al., J. Surg. Res (1989) 28:151-170. To compensate for insensible fluid loss, animals were infused with isotonic saline at 3.3 ml/kg/hr for 12 hours via the brachial vein 30 minutes or 240 minutes, respectively, after the administration of bacteria. LACI was administered at a loading dose of 700 $\mu$g/kg for 15 minutes and a continuous infusion of LACI at 10 $\mu$g/kg/min was given for an additional 525 minutes (counting from start of bacterial infusion, which was defined as time zero). To deliver the same total LACI dose per baboon, animals treated at +240 minutes received a loading dose of 2.8 $\mu$g/kg for fifteen minutes and simultaneously received a continuous infusion of LACI at 10 $\mu$g/kg/min for 480 min.

*E. coli* 086:K61H were used to inoculate tryptic soy broth agar, and viability counts of the inoculum were determined by standard dilution techniques. At time zero, baboons received an infusion of $\geq$ 4.5 x 10$^{10}$ live bacteria per kg body weight (4 ml/kg), administered through a percutaneous catheter in the right cephalic vein by continuous infusion for 2 hours.

The femoral artery and one femoral vein were cannulated aseptically to measure mean systemic arterial pressure, obtain blood samples and for antibiotic administration. Gentamicin was given (9 mg/kg i.v.) at the end of *E. coli* infusion, i.e., at T + 120 for 30 minutes and then 4.5 mg/kg at T + 360 and T + 540 minutes for 30 min. Gentamicin (4.5 mg/kg IM) was then given at the end of the experiment and once daily for 3 days.

Animals were maintained under anesthesia and monitored continuously for 12 hours. Blood samples were collected hourly for hematology, clinical chemistry, cytokines (TNF, IL-6), and LACI determinations. Similarly, respiration rate, heart rate, mean systemic arterial pressure and temperature were monitored hourly. Animals were continuously observed for the first 30 hours of the experiment. Those surviving 7 days were considered permanent survivors and were subsequently euthanized with sodium pentobarbital for necropsy at the 8th day.

Ten baboons (5 LACI treated and 5 excipient controls) were intravenously administered 2 hour lethal infusions of *E. coli.* Table 1 shows that LACI rescued five of five *E. coli* treated baboons who became permanent survivors. The mean *E. coli* dosage of the LACI treated was $5.7 \times 10^{10}$ CFU/kg and all animals survived more than 7 days. The mean *E. coli* dosage of the excipient control group was $5.5 \times 10^{10}$ CFU/kg and the mean survival was 39.9 hours (Table 1). The mean weight of the excipient control group was 8.4 kg (range 5.9 to 12.1 kg) and that of the LACI treated was 6.8 kg (range 5.2 to 8.0 kg). Two females and three males composed the excipient control group, while the LACI treated group consisted of five males. There was no difference in the mean dose of *E. coli* administered to each group (p > 0.05) nor in the animals' weights (p > 0.05).

LACI treated baboons moved about the cage energetically, consumed some food and drank water normally within 24 hours of receiving lethal *E. coli* ($LD_{100}$). The excipient control baboons, however, were very lethargic, appeared to have difficulty breathing and exhibited multiple petechiae over their bodies indicating the occurrence of DIC in the dermal microvasculature.

Coagulation and Hematological Responses to LACI Administration at +30 Minutes

To determine the mechanism by which LACI protected the bacterially infected baboons we measured selected physiologic parameters associated with coagulation, clinical chemistries and the inflammatory response. Figure 6 shows that many of the coagulopathies associated with the bacterial infection were inhibited and/or attenuated in the LACI treated baboons. Fibrinogen levels in excipient control animals dropped by approximately 80% by 3 hours, while the LACI treated baboons experienced only a 20% drop (p < 0.0001). Similarly, the rise in fibrin degradation products at 240 and 720 minutes, as a marker of fibrinogen consumption, was not evident in the LACI treated animals as compared to the controls (p < 0.05).

Activated partial thromboplastin time (APTT) and prothrombin time (PT) were extremely prolonged at times beyond 4 hours in the excipient controls (Figure 6). APTT increased from 37 to 208 and then to 226 seconds while PT increased from 14 to 58 and then to 137 seconds, at 4 and 12 hours, respectively. In contrast, APTT increased from 32 to 45 to 60 seconds at 4 and 12 hours, respectively, and PT increased from 15 to 18 seconds to 22 seconds at 4 and 12 hours, respectively, in the LACI treated baboons (p < 0.05).

A gradual drop in platelet cell concentration was noted in the excipient controls and in the LACI treated baboons over the 12 hour observation period (Figure 6). LACI treatment, however, retards the drop and is most apparent at $\geq$ 4 hours. The mean platelet concentration of the control group at four, six and twelve hours were $102.8 \pm 26$, $69 \pm 20$ and $43 \pm 5.0$. In contrast, the mean platelet concentration of the LACI treated group at the same times were $249 \pm 44$, $236 \pm 35$, and $153 \pm 31$, respectively.

Despite the lack of visible hemolysis in the LACI treated plasma samples, the hematocrit decreased with time and was lower at 12 hours in the experimental (treated) group, $36 \pm 2\%$, as compared to the control group, $44 \pm 2.\%$ (p < 0.05). Furthermore, the mean 7 day hematocrit value of the survivors was also low as compared to baseline: $28 \pm 1\%$ versus $42 \pm 0\%$.

Consistent with the hematocrit results the red blood cell concentration dropped only slightly over the initial 12 hours in both the control ($4.94 \pm .21$ to $4.4 \pm 0.11$) and LACI treated groups ($5.20 \pm 0.10$ to $4.88 \pm 0.17$), and a low ($3.42 \pm .2 \times 10^6$) red cell concentration was observed in the survivors.

Leukopenia occurred to the same degree in the LACI treated and control group, the lowest values ($\sim 1.48 \times 10^3/\mu l$) recorded at 2 hours; however, the white blood cell concentration was found elevated at 7 days in the survivors with a mean of $19.2 \pm 3.5$ as compared to the base line of $9.0 \pm 1.5 \times 10^3/\mu l$.

Clinical Responses to LACI Administration at +30 Minutes

Respiration and heart rate increased in both groups. Respiration rate rose quickly after the start of the bacterial infusion and remained elevated for the 12 hour period. Similarly, heart rate increased dramatically, from 120 beats/min to 200 beats/min, within the first two hours of *E. coli* infusion and remained elevated during the 12 hours.

Mean systolic arterial pressure (MSAP) and temperature equally declined in the LACI treated and control groups. A dramatic decrease in MSAP was observed at the end of the bacterial infusion. MSAP declined from $107 \pm 5$ mm Hg to $69 \pm 5$ at two hours and then gradually returned to $93 \pm 11$ by 10 to 12 hours in the control group. Similarly, MSAP declined from $115 \pm 9$ to $74 \pm 3$ at 2 hours and rose to $85 \pm 7$ from 6 hours on to 12 hours. The ten baboons had a decreased temperature response to the *E. coli* infusion. The mean excipient control temperature at the start of the experiment was $37.3 \pm 0.1°C$ and declined slowly to $34.7 \pm 2.2°C$ at 12 hours. The mean LACI treated temperature was initially $37.0 \pm 0.3°C$ and changed minimally over the 12 hours where it was $36.9 \pm 0.2°C$.

Blood Chemistries

Table 2 summarizes clinical chemistries of the *E. coli* infected and treated ten baboons. Increases in serum creatinine, total bilirubin, uric acid, lactic acid, triglycerides, anion gap, chloride and sodium were measured at 12 hours. The magnitude of the increases, however, was lower in the LACI treated animals than the excipient controls ($p < 0.05$). Changes in the concentrations of the following parameters were observed: albumin, alkaline phosphatase, AST, BUN, calcium, cholesterol, CK, carbon dioxide, cortisol, potassium, lactic dehydrogenase, phosphorous, SGPT and total protein. Their increases or decreases in concentration were not affected by the LACI treatment ($p > 0.05$). However, the mean concentrations of albumin, urea nitrogen (BUN) and lactate did not return to baseline values in the LACI treated animals (i.e. the survivors) at 7 days. Specifically, albumin concentrations were $2.7 \pm 0.2$ at 7 days as compared to $3.7 \pm 0.1$ at the start of the experiments. Thus albumin was reduced by about 25%. Similarly, serum values of urea nitrogen (BUN) at 7 days was $13.8 \pm 2.1$ versus $29.6 \pm 3.9$ at the beginning of the experiment. Finally, lactate concentrations were increased by about 3-fold in the survivors. The mean baseline lactate concentrations of these animals was $1.7 \pm 0.5$ meq/L at the start of the procedure and increased to $5.7 \pm 1.2$ meq/L at 7 days.

Increases in glucose concentration were observed within two hours in both groups ($p < 0.05$). Mean values fell gradually beyond the initial increase but remained consistently higher in the LACI treated animals ($p < 0.05$) until 12 hours. Increases in arterial pH occurred in both groups.

TNF and IL-6 Levels

Plasma TNF concentrations were elevated in both the excipient group and LACI treated baboons. Consistent with our previous studies (Creasey et al., Circ. Shock (1991) 33:84-91), peak TNF levels were at 120 min, i.e. at the end of *E. coli* infusion. LACI treatment did not appear to affect the rise in serum TNF concentrations nor the kinetics of its release (Table 3). Plasma IL-6 concentrations also increased with time in the excipient control group, where IL-6 levels started at 26-39 picograms and rose to 100-200 nanograms beyond four hours (Table 4). Interestingly, plasma IL-6 concentrations in the LACI treated animals were lower than those of the control group, especially at and beyond four hours. IL-6 concentrations were about 20-fold lower in the LACI treated than the excipient controls at 12 hours ($p < .05$).

Administration of LACI at +240 Minutes

To determine the time beyond which LACI may no longer be effective in attenuating the *E. coli* shock, we delayed the administration of LACI to two hours after the end of the bacterial infusion. Fibrinogen consumption and the generation of fibrin degradation products were to be clearly evident at four hours. Table 5 shows that the mean *E. coli* dosage of the excipient control group in this series of experiments was $5.68$ ($\pm 2.6$) x $10^{10}$ CFU/kg and the mean survival time of $28.2 \pm 9.6$ hours. The mean *E. coli* dosage of the LACI group was $5.43$ ($\pm 0.19$) x $10^{10}$ CFU/kg and the mean survival time of $99 \pm 29$ hours. Two of the five LACI treated animals were 7 day survivors ($p < 0.05$). There was no difference in the mean weight or *E. coli* dosage administered to each of the above groups ($p > 0.05$).

Biological and Biochemical Effects of Administration of LACI at +240 Minutes

The administration of LACI two hours after the end of the two hour bacterial infusion was effective in slightly attenuating the coagulopathic response as evident by decreases in FDP levels, and prothrombin time at $\geq 12$ hours. Consistent with +30 minutes, IL-6 levels were two-fold lower in the LACI treated baboons than their excipient counterparts at 12 hours. No significant differences in fibrinogen concentrations, APTT and platelet cell concentration were noted at 12 hours between the excipient control and the LACI treated baboons. However, fibrinogen levels at day 7 in the two animals that survived were slightly elevated; FDP, APTT and PT values were back close to normal while platelet cell concentrations were normal in one (435) and lower in the other (97).

Although the red blood cell count and hematocrit fluctuated slightly over time in both groups during the first 12 hours, the two survivors had lower hematocrits at day 7 (35 and 19%) as compared to the start of the procedure (43 and 41 %). Similarly, red blood cell concentration was 4.0 and 2.7 x $10^6$/mm$^3$ on day 7 versus 4.7 and 4.5 x $10^6$/mm$^3$ at day 0.

Clinical chemistries were measured for the ten baboons comprising the plus four hour study as we had performed for the plus thirty minute study. We observed minimal differences between the excipient control and LACI treated baboons at twelve hours. However, consistent with the plus 30 minute study, lactate levels were higher in the LACI treated than the controls (p < 0.05) at 12 hours and remained elevated in the two that survived 7 days (13.2 and 4.0 mg/dl versus 0.5 and 0.6 mg/dl at time 0). In contrast, uric acid levels were slightly lower in the LACI treated group than the controls at 12 hours and returned to normal levels in the two LACI treated survivors.

Similar to the plus 30 minute study, all the animals treated at 240 minutes experienced leukopenia, and a gradual but small rise in WBC count over the twelve hours. Furthermore, the two 7 day surviving LACI treated baboons had elevated WBC counts (12.5 and 21.8 x $10^3$ cells/mm$^3$) at day 7 as compared to 5.1 and 8.0 x $10^3$ cells/mm$^3$ at time zero; this trend is similar to that observed in the survivors of the baboons treated at +30 minutes with LACI.

Pathological Results

Post-mortem examinations were conducted on all baboons. Surveillance of animals was continuous for the first 36 hours; consequently tissues were removed for analysis within minutes after death thereby avoiding post-mortem autolytic changes. Lungs, liver, adrenals, kidneys, spleen, and gall bladder were target organs of the *E. coli* bacterial infusion. Specifically, animals that received excipient + *E. coli* suffered from severe congestion, hemorrhage, fibrin deposition, edema and massive accumulation of leukocytes in the lungs and liver, severe congestion of medullary sinusoids in the spleen and significant evidence of tubular necrosis and thrombosis within the kidneys and severe cortical congestion in the adrenals. Organs not affected by *E. coli* were stomach, heart, pancreas and small and large intestines. LACI protected the liver, adrenals, kidneys, spleen and gall bladder in which only mild to no pathology were observed. The degree of protection was slightly diminished in the lungs, in which moderate vascular congestion, and mild leukocyte accumulation were observed.

Results from the present study demonstrated that LACI rescued one hundred percent of the baboons given LD$_{100}$ doses of *E. coli* when administered thirty minutes after the start of the bacterial infusion when more than 1 x $10^{10}$ organisms/kg had already been introduced into the blood of the baboons. In addition, LACI rescued forty percent of the baboons when given two hours after the end of the bacterial infusion i.e. when greater than 5 x $10^{10}$ organisms/kg had been infused and many of the baboons' host defense mechanisms had been triggered for two hours.

TNF levels peaked at the end of the *E. coli* infusion i.e. at two hours, while IL-1β and IL-6 levels started to appear (Creasey et al., Circ. Shock (1991) 33:84-91); the decline and consumption of fibrinogen and generation of fibrin degradation products become more easily detectable between three and four hours (De Boer, J.P. et al., Circ. Shock (In press 1992)). This study shows that LACI could prevent, slow down and even reverse the consumption of fibrinogen, when administered as late as four hours after the start of a lethal bacterial infusion.

In addition to attenuating coagulation, LACI attenuated the degree of cell injury (creatinine, uric acid, lactic acid) and metabolic acidosis (anion gap, chloride and sodium) so clearly evident in the controls. Consistent with the decreased serum levels of many of these markers of hypoxia, acidosis and cell injury, LACI afforded remarkable morphological protection to kidneys, adrenals, liver, spleen and the lungs from pathological changes. The efficacy of LACI in baboons challenged with lethal *E. coli* shows gram-negative shock is an acute inflammatory disease of the vascular endothelium and that significant benefit is achieved by transiently protecting the endothelium from insults associated with gram-negative bacteria.

Our previous studies have shown that within the first 30 minutes of the bacterial infusion, the PMN leukocyte concentration in circulating blood fell sharply (Taylor et at, Colloquium Mosbach Molecular Aspects of Inflammation (1991) Springer Verlag, Berlin Heidelberg, pp. 277-288), thrombin-antithmmbin (TAT) complexes, tissue plasminogen activator/plasminogen activator inhibitor (t-PA/PAI) and plasmin anti plasmin (PAP) complexes had started to appear (De Boer, J.P. et al., Circ. Shock (1992) In press), and the activation of the complement cascade in lethal *E. coli* challenge was clearly evident (De Boer. J.P. et al., submitted). LACI treatment resulted in the prevention of tubular necrosis and glomerular thrombosis in the kidneys; cortical congestion, hemorrhage, necrosis and leukocyte accumulation in the adrenals; prevention of vascular congestion and accumulation of leukocytes in the liver; prevention of medullary congestion, hemorrhage and necrosis in the spleen; and fibrin thrombi deposition and edema formation in the lungs. LACI significantly attenuated leukocyte influx and vascular congestion in the lungs. The two baboons that received LACI at four hours and survived seven days showed a very similar prevention of pathological changes as those described above. However, there was some mild edema and fibrin present in alveolar sacs of the lungs with moderate leukocyte accumulation and vascular congestion. There was no evidence of multiple organ failure in any of the LACI treated baboons that survived seven days. This degree of protection is remarkable and unexpected given the delayed administration of LACI and the massive bacterial challenge afforded to the baboons.

The LACI-treated, *E. coli* challenged, 7 day survivors demonstrated a lower red blood cell concentration and an increase in leukocyte concentration. Histological examination did not reveal the occurrence of hemorrhage in any tissue. Thus the lower hematocrit may be attributed either to hemodilution or to the slow generation of erythrocytes in the bone marrow. LACI toxicology studies with uninfected baboons may be necessary to resolve this matter.

The decreased IL-6 levels observed in the *E. coli* challenged and LACI treated baboons in the present study show was unexpected and suggest that LACI either directly or indirectly exhibits an effect on the inflammatory response. Thus, in addition to its anticoagulant activity, a physiologic role of LACI is useful in the modulation of the interaction of the coagulation pathway with various participants of the immune system.

Table 1

| Weight, Sex, *E. coli* Dose and Survival Times of Control and LACI* Treated Baboons at +30 min** | | | | |
|---|---|---|---|---|
| | Weight (kg) | Sex | Mean Dose *E. coli* (CFU/kg x $10^{10}$) | Survival Time (hrs) |
| Control (*E. coli* + excipient control) | | | | |
| 26 | 12.1 | M | 5.71 | 46 |
| 27 | 9.8 | F | 5.60 | 52.5 |
| 32 | 6.4 | F | 5.23 | 9.7 |
| 37 | 7.7 | M | 5.26 | 30.5 |
| 41 | 5.9 | M | 5.70 | 60.5 |
| Mean ($\pm$ SE) | 8.4 $\pm$ 1.1 | | 5.50 $\pm$ 0.11 | 39.9 $\pm$ 9.0 |
| Experimental (*E. coli* + LACI) | | | | |
| 29 | 8.0 | M | 4.84 | >168 |
| 30 | 7.5 | M | 5.22 | >168 |
| 31 | 7.3 | M | 6.05 | >168 |
| 38 | 5.2 | M | 6.21 | >168 |
| 40 | 6.1 | M | 6.15 | $\geq$168 |
| Mean ($\pm$ SE) | 6.8 $\pm$ 0.5 | | 5.69 $\pm$ 0.28 | 168 $\pm$ 0.0 |

*LACI - Tissue Factor Pathway Inhibitor
**LACI administered 30 min after the onset of a 2 hr infusion of *E. ccli*

Table 2

| Clinical Chemistry Summary of LACI Treated and Control Baboons at +30 min** | | | | | |
|---|---|---|---|---|---|
| | Control (Mean ± STD error) | | LACI (Mean ± STD error) | | |
| | T 0 | T + 12 hrs | T 0 | T + 12 hrs | + 7 days |
| **p < 0.05:** | | | | | |
| Creat (mg/dL) | 0.64 ± 0.05 | 2.68 ± 0.27 | 0.64 ± 0.09 | 0.92 ± 0.07 | 0.48 ± .07 |
| T Bili (mg/dL) | 0.16 ± 0.02 | 1.35 ± 0.33 | 0.14 ± .02 | 0.30 ± 0.11 | 0.20 ± 0.05 |
| Uric Acid (mg/dL) | 0.38 ± 0.07 | 0.93 ± 0.18 | 0.50 ± 0.0 | 0.50 ± 0.00 | 0.32 ± 0.07 |
| Lactate (mEq/L) | 0.94 ± 0.38 | 6.05 ± 0.59 | 1.74 ± 0.47 | 4.10 ± 0.34 | 5.70 ± 1.21 |
| Triglycerides (mg/dL) | 64 ± 7 | 283 ± 19 | 101 ± 25 | 161 ± 28 | 130 ± 42 |
| Anion GAP (mEq/L) | 13.4 ± 0.75 | 19.25 ± 0.63 | 11.2 ± 1.24 | 11.25 ± .25 | 12.75 ± 1.03 |
| Cl (mEq/L) | 107.68 ± 3.73 | 109.58 ± 1.16 | 105.76 ± 0.52 | 117.62 ± 1.08 | 100.56 ± 6.61 |
| Na (mEq/L) | 150.58 ± 4.81 | 149.50 ± 0.65 | 146.04 ± 1.31 | 153.0 ± 1.0 | 142.26 ± 8.51 |
| **p > 0.05** | | | | | |
| Alb (g/dL) | 3.82 ± 0.27 | 2.90 ± 0.33 | 3.66 ± 0.13 | 2.8 ± 0.06 | 2.68 ± 0.22 |
| Alk phosp. (IU/L) | 827 ± 59 | 949 ± 62 | 933 ± 68 | 1032 ± 121 | 937 ± 117 |
| AST (U/L) | 40 ± 3 | 1531 ± 783 | 45 ± 5 | 710 ± 484 | 68 ± 8 |
| BUN (mg/dL) | 19.4 ± 1.9 | 39.0 ± 4.1 | 29.6 ± 3.9 | 34.0 ± 3.2 | 13.8 ± 2.1 |
| CA (mg/dL) | 9.9 ± 0.4 | 7.1 ± 0.3 | 10.3 ± 0.2 | 7.9 ± 0.2 | 9.1 ± 0.5 |
| Chol (mg/dL) | 126 ± 10 | 94 ± 7 | 130 ± 3 | 86 ± 3 | 135 ± 16 |
| CK (U/L) | 604 ± 130 | 5979 ± 1705 | 795 ± 348 | 5594 ± 732 | 289 ± 79 |
| $CO_2$ (mEq/L) | 29.6 ± 2.0 | 20.4 ± 1.7 | 29.2 ± 0.9 | 23.7 ± 0.6 | 28.2 ± 1.9 |
| Cortisol (μg/dL) | 48.4 ± 7.9 | 120.2 ± 21.1 | 48.2 ± 12.2 | 110.5 ± 16.2 | 28.5 ± 2.6 |
| K (mEq/L) | 3.90 ± 0.14 | 4.75 ± 0.44 | 3.86 ± 0.14 | 4.32 ± 0.10 | 3.62 ± 0.25 |
| LDH (IU/L) | 311 ± 46 | 3956 ± 1112 | 317 ± 40 | 1819 ± 621 | 433 ± 58 |
| Phos (mg/dL) | 5.94 ± 0.69 | 8.28 ± 0.59 | 4.78 ± 0.34 | 7.66 ± 0.67 | 3.96 ± 0.55 |
| SGPT (IU/L) | 69 ± 23 | 936 ± 507 | 49 ± 9 | 366 ± 281 | 101 ± 11 |
| Total Protein (g/dL) | 7.00 ± 0.36 | 5.88 ± 0.35 | 6.62 ± 0.21 | 5.36 ± 0.18 | 5.92 ± 0.36 |

*LACI = Tissue Factor Pathway Inhibitor
**LACI administered 30 min after the onset of a 2 hr infusion of *E. ccl*

Table 3

| Individual Animal IL-6 Levels (ng/ml) LACI Administration at +30 min | | | | | | |
|---|---|---|---|---|---|---|
| | T 0 | +30 | +120 | +240 | +360 | +720 |
| **Control (*E. coli* + excipient control)** | | | | | | |
| 26 | .034 | .027 | 21.5 | 102.3 | 347.2 | 468.5 |
| 27 | .018 | .047 | 27.6 | 58.4 | 88.7 | 31.1 |
| 32 | .010 | .020 | 35.6 | 217.6 | 321.7 | NT |
| 37 | .038 | .048 | 36.7 | 97.6 | 196.9 | 183.2 |
| 41 | .028 | .052 | 32.3 | 101.7 | 100.4 | 63.4 |
| Mean ± SE | .03 | .04 | 30.7 | 116 | 211 | 187 |
| | ± .01 | ± .01 | ± 2.8 | ± 26.8 | ± 57.5 | ±63.4 |
| **Experimental (*E. coli* + LACI)** | | | | | | |
| 29 | NT | NT | 30.0 | 57.3 | 50.8 | 12.5 |
| 30 | .150 | .639 | 64.2 | 51.1 | 26.1 | 7.1 |
| 31 | .013 | .030 | 31.8 | 48.0 | 36.7 | 10.7 |
| 38 | .034 | .049 | 16.5 | 42.7 | 30.6 | 6.4 |
| 40 | .059 | .058 | 17.3 | 24.8 | 23.7 | 11.3 |
| Mean ± SE | .06 | .19 | 32.0 | 44.8 | 33.6 | 9.6 |
| | ± .03 | ±.129 | ± 8.7 | ± 5.5 | ± 4.8 | ± 1.2 |

NT = Not Tested

Table 4

| Weight, Sex, *E. coli* Dose and Survival Times of Control and LACI* Treated Baboons at +240 min** | | | | |
|---|---|---|---|---|
| | Weight (kg) | Sex | Mean Dose *E. coli* (CFU)/kg x $10^{10}$) | Survival Time (hrs) |
| **Control (*E. coli* + excipient control)** | | | | |
| 33 | 6.8 | M | 6.22 | 63.5 |
| 45 | 7.7 | F | 6.29 | 18.0 |
| 46 | 9.1 | M | 4.94 | 32.5 |
| 47 | 6.6 | M | 5.26 | 9.0 |
| 48 | 7.1 | M | 5.70 | 18.0 |
| Mean (± SE) | $\overline{7.5} \pm 0.5$ | | $\overline{5.68} \pm .26$ | $\overline{28.2} \pm 9.6$ |
| **Experimental (*E. coli* + LACI)** | | | | |
| 34 | 5.2 | M | 5.65 | 58 |
| 35 | 7.3 | M | 5.62 | >168 |
| 36 | 6.8 | M | 4.84 | >168 |
| 44 | 9.1 | M | 5.87 | 69 |
| 49 | 7.5 | F | 5.17 | 35 |
| Mean (± SE) | $\overline{7.2} \pm 0.6$ | | $\overline{5.43} \pm 0.19$ | $\overline{99.6} \pm 28.5$ |

*LACI = Tissue Factor Pathway Inhihitor
** 240 min after the onset of a 2 hr infusion of *E. ccli*

## Claims

1. Use of lipoprotein associated coagulation inhibitor (LACI) or a fragment or hybrid thereof which maintains anti-inflammatory activity in the manufacture of a medicament for use in a prophylactic or therapeutic method of treating acute or chronic inflammation.

2. Use according to claim 1 wherein the acute or chronic inflammation is sepsis or septic shock.

3. Use according to claim 2 of LACI and an additional agent in the manufacture of a combined medicament for use in the treatment of sepsis.

4. Use according to claim 3, wherein the additional agent is an antibiotic, a monoclonal antibody, a cytokine or a complement inhibitor.

5. Use according to any one of claims 1 to 4, wherein LACI is chemically conjugated to a polymer consisting essentially of polyethylene glycol (PEG) or polyoxyethylated glycerol (POG).

6. Use according to any one of the preceding claims wherein LACI is present in an amount so as to provide a dose between 1µg/kg and 20µg/kg.

7. Use according to any one of the preceding claims wherein LACI is present in a form suitable for administration as a bolus dose or a continuous infusion.

**Patentansprüche**

1. Verwendung des Lipoprotein-assoziierten-Koagulations-Inhibitors (LACI) oder eines Fragments oder Hybrids davon mit beibehaltener anti-inflammatorischer Aktivität zur Herstellung eines Medikaments zur Verwendung in einem prophylaktischen oder therapeutischen Verfahren zur Behandlung einer akuten oder chronischen Entzündung.

2. Verwendung nach Anspruch 1, wobei die akute oder chronische Entzündung eine Sepsis oder ein septischer Schock ist.

3. Verwendung nach Anspruch 2 von LACI und einem zusätzlichen Mittel zur Herstellung eines kombinierten Medikaments zur Verwendung bei der Behandlung einer Sepsis.

4. Verwendung nach Anspruch 3, wobei das zusätzliche Mittel ein Antibiotikum, ein monoclonaler Antikörper, ein Cytokin oder ein Komplement-Inhibitor ist.

5. Verwendung nach einem der Ansprüche 1 bis 4, wobei LACI chemisch mit einem Polymer konjugiert ist, das im wesentlichen aus Polyethylenglykol (PEG) oder polyoxyethyliertem Glycerin (POG) zusammengesetzt ist.

6. Verwendung nach einem der vorhergehenden Ansprüche, wobei LACI in einer Menge vorhanden ist, mit der eine Dosis zwischen 1 µg/kg und 20 µg/kg bereitgestellt werden kann.

7. Verwendung nach einem der vorhergehenden Ansprüche, wobei LACI in einer Form vorliegt, die geeignet ist für eine Verabreichung als eine Bolus-Dosis oder eine kontinuierliche Infusion.

**Revendications**

1. Utilisation de l'Inhibiteur de Coagulation Associé aux Lipoprotéines (LACI) ou un fragment ou un hybride de celui-ci qui maintient une activité anti-inflammatoire, dans la fabrication d'un médicament destiné à une utilisation dans une méthode prophylactique ou thérapeutique de traitement d'une inflammation aiguë ou chronique.

2. Utilisation selon la revendication 1 dans laquelle l'inflammation aiguë ou chronique est une septicémie ou un choc septique.

3. Utilisation selon la revendication 2 du LACI et d'un agent supplémentaire dans la fabrication d'un médicament combiné destiné à une utilisation dans le traitement d'une septicémie.

4. Utilisation selon la revendication 3, dans laquelle l'agent supplémentaire est un antibiotique, un anticorps monoclonal, une cytokine ou un inhibiteur du complément.

5. Utilisation selon l'une quelconque des revendications 1 à 4, dans laquelle LACI est chimiquement conjugué à un polymère constitué essentiellement de polyéthylèneglycol (PEG) ou de glycérol polyoxyéthylénique (POG).

6. Utilisation selon l'une quelconque des revendications précédentes dans laquelle LACI est présent en une quantité apte à fournir une dose entre 1 µg/kg et 20 µg/kg.

7. Utilisation selon l'une quelconque des revendications précédentes dans laquelle LACI est présent sous une forme appropriée à une administration comme dose d'embol ou perfusion continue.

**FIG. 1**

INTRINSIC PATHWAY          EXTRINSIC PATHWAY

XII → XIIa

XI → XIa   VIIIa          VIIa → VII

IX → IXa                  TISSUE FACTOR

X → Xa ← X

COMMON PATHWAY
Va →

PROTHROMBIN → THROMBIN

FIBRINOGEN → FIBRIN

XIIIa →

CLOT

CIRCULATING THROMBI

BACTERIA LPS

ACTIVATION OF COAGULATION

EVENTS LEADING TO THROMBOSIS

EVENTS LEADING TO BLEEDING

THROMBOTIC OCCLUSION OF MICROCIRCULATION OF ALL ORGANS → FIBRINOLYSIS IN THE MICROCIRCULATION → CIRCULATING FIBRIN DEGRADATION PRODUCTS + CONSUMPTION OF PLATELETS AND COAGULATION PROTEINS

SIGNS OF MICROVASCULAR THROMBOSIS          SIGNS OF HEMORRHAGIC DIATHESIS

EP 0 643 585 B1

# FIGURE 2

# FIGURE 3

FIGURE 4

EP 0 643 585 B1

FIGURE 5a

FIGURE 5b

EP 0 643 585 B1

FIGURE 6a

FIGURE 6b

FIGURE 6c

FIGURE 6d

FIGURE 6e

FIGURE 6f

FIGURE 6g

FIGURE 6h

26